# EUROPEAN PATENT SPECIFICATION

(11) **EP 3 047 812 B1**
(45) Date of publication and mention of the grant of the patent: **01.01.2020**
(21) Application number: 16155903.4
(22) Date of filing: 22.11.2010
(51) Int. Cl.: A61B 17/70

(54) **POLYAXIAL BONE ANCHORING DEVICE**
POLYAXIALE KNOCHENVERANKERUNGSVORRICHTUNG
DISPOSITIF D'ANCRAGE D'OS POLYAXIAL

(43) Date of publication of application: 27.07.2016
(62) Divisional of application: 10192079.1
(73) Proprietor: Biedermann Technologies GmbH & Co. KG, 78166 Donaueschingen (DE)
(72) Inventor: BIEDERMANN, Lutz, 78048 VS-Villingen (DE); POHL, Gerhard, 78112 St. Georgen (DE)
(74) Representative: Prüfer & Partner mbB Patentanwälte · Rechtsanwälte

(56) References cited:
- EP-A1- 2 221 012
- US-A- 5 672 176
- US-A- 5 681 319
- US-A1- 2009 105 770

## Description

The invention relates to a polyaxial bone anchoring device for anchoring a stabilization rod in a bone or in a vertebra. The bone anchoring device includes an anchoring element, a receiving part for receiving a head of the bone anchoring element and for receiving a stabilization rod to be connected to the anchoring element. The anchoring element is pivotably connected to the receiving part and can be locked at an angle by exerting pressure onto the head via a pressure element that is arranged in the receiving part. The pressure element and the receiving part are configured to cooperate in such a way that the pressure element frictionally clamps the head to maintain a desired angular position before locking.

US 5,716,356 describes a polyaxial bone screw including a screw element and a receiving part which is pivotably connected to the screw element and a pressure element to exert pressure onto the head of the screw element to lock the angle between the screw element and the receiving part. The receiving part has a U-shaped channel for receiving a stabilization rod. The pressure element comprises a cylindrical recess, which is to be aligned with the U-shaped channel to receive the rod therein. In order to hold the pressure element in a position aligned with the U-shaped channel, the position of the pressure element is fixed by crimping through bores provided in the receiving part.

When the head of the bone anchoring element is freely pivotable with respect to the receiving part before locking the head in a final angular position, the alignment of the receiving part and the insertion of the rod may be difficult in more complex clinical applications, for example, when a multitude of bone anchors have to be connected to the rod.

US 7,604,656 describes a fastener engageable with a bone portion to connect a longitudinal member to the bone portion. The housing that receives the fastener also receives a spacer, which is engageable with the fastener and the longitudinal member. In one embodiment, the spacer is urged by a pin member into frictional engagement with the fastener and with the housing.

US 2004/0267264 A1 describes a polyaxial fixation device, wherein the polyaxial bone screw includes an engagement member that is adapted to provide sufficient friction between the spherical head and the receiver member to enable the shank to be maintained in a desired angular orientation before locking the spherical head within the receiver member. The engagement member is realized, for example, by a snap ring around the head or by spring members provided at the compression cap to frictionally engage the spherical head or by a slot provided in the compression cap.

EP 2 221 012 A1 discloses a receiving part for receiving a rod for coupling the rod to a bone anchoring element. The receiving part includes a body with a channel for receiving the rod thereby forming two legs, an accommodation space for accommodating a head of a bone anchoring element and a pressure element located at least partially in the accommodation space. The pressure element has a U-shaped recess for receiving the rod and an outer wall in which elongate recesses are formed. On each leg of the receiving part a bore extends through the leg for receiving pins, respectively. When inserted into the bores, the pins may engage the elongate recesses of the pressure element to prevent the pressure element from escaping through an open upper end of the receiving part.

US 5,681,319 A discloses a locking tool configured to engage a bone screw comprising a seat part, a pressure member and a screw member. The pressure member has an outer diameter selected so as to allow a sliding motion of the pressure member within a bore of the seat part. Lateral side walls of the pressure member are formed with blind holes, which are aligned with corresponding pocket bores within outer walls of the seat part, when the pressure member rests on a head of the screw member in an assembled state.

It is an object of the invention to provide a polyaxial bone anchoring device, which allows an improved handling during surgery and which can be manufactured in a simple manner.

The object is solved by a polyaxial bone anchoring device according to claim 1, 2, 12 or 13. Further developments are given in the dependent claims.

With the polyaxial bone anchoring device a temporary clamping of the head in a desired angular position with respect to the receiving part without locking the head can be achieved. This allows to maintain the receiving part in an adjustable angular position. In this condition, the pressure element exerts a preload onto the head in which the head is not locked but prevented from freely pivoting. When the head is temporarily clamped, the alignment of the receiving part with respect to the rod and the insertion of the rod is facilitated, in particular in a situation in which a multitude of bone anchors have to be connected to the rod.

When the rod is already inserted into the receiving part, adjustments of the rod are still possible without completely loosening the head.

The polyaxial bone anchoring device comprises only few parts which are of simple design. The mechanism to frictionally maintain the head before locking it is free from any spring members or portions. This facilitates the manufacturing of the polyaxial bone anchoring device. Furthermore, existing receiving parts and pressure elements can be used without having to redesign their shape. It is possible to simply change the location of the crimp bores.

The amount of preload exerted onto the head by the pressure member can be exactly predefined in a simple manner by selecting the position and shape of the crimp bores. The polyaxial bone anchoring device is provided to the surgeon in a pre-assembled manner, in which the pressure element is axially and rotationally fixed to such an extent that it can not fall out or be rotated out of its aligned position. This allows a safe handling by the surgeon.

The receiving part and the pressure element can be manufactured in series at low costs.

Further features and advantages of the invention will become apparent from the description of embodiments by means of the accompanying drawings.

In the drawings:
- Fig. 1: shows a perspective exploded view of the polyaxial bone anchoring device according to a first embodiment.
- Fig. 2: shows the polyaxial bone anchoring device of Fig. 1 in an assembled state.
- Fig. 3: shows a cross-sectional view of the polyaxial bone anchoring device in an assembled state before final locking of the head.
- Fig. 4a: shows a cross-sectional view of the polyaxial bone anchoring device before provisionally fixing the pressure element in the receiving part.
- Fig. 4b: shows an enlarged portion of Fig. 4a.
- Fig. 5a: shows a cross-sectional view of the polyaxial bone anchoring device in the preassembled state after provisionally fixing of the pressure element in the receiving part.
- Fig. 5b: shows an enlarged portion of Fig. 5a.
- Fig. 6: shows a cross-sectional view of a tool for provisionally fixing the pressure element in the receiving part.
- Fig. 7: shows an enlarged portion of Fig. 6.
- Fig. 8: shows a cross-sectional view of a modified embodiment of the polyaxial bone anchoring device before locking of the head.
- Fig. 9: shows a cross-sectional view of a further modified embodiment of the polyaxial bone anchoring device in a state before locking of the head.
- Fig. 10: shows a perspective exploded view of a second embodiment of the polyaxial bone anchoring device.
- Fig. 11: shows an enlarged cross-sectional view of a portion of the pressure element of the bone anchoring device of Fig. 10.
- Fig. 12: shows a cross-sectional view of the polyaxial bone anchoring device of the second embodiment before provisionally fixing the pressure element in the receiving part.
- Fig. 13: shows a cross-sectional view of the polyaxial bone anchoring device of Fig. 12 in the pre-assembled state after provisionally fixing of the pressure element in the receiving part.

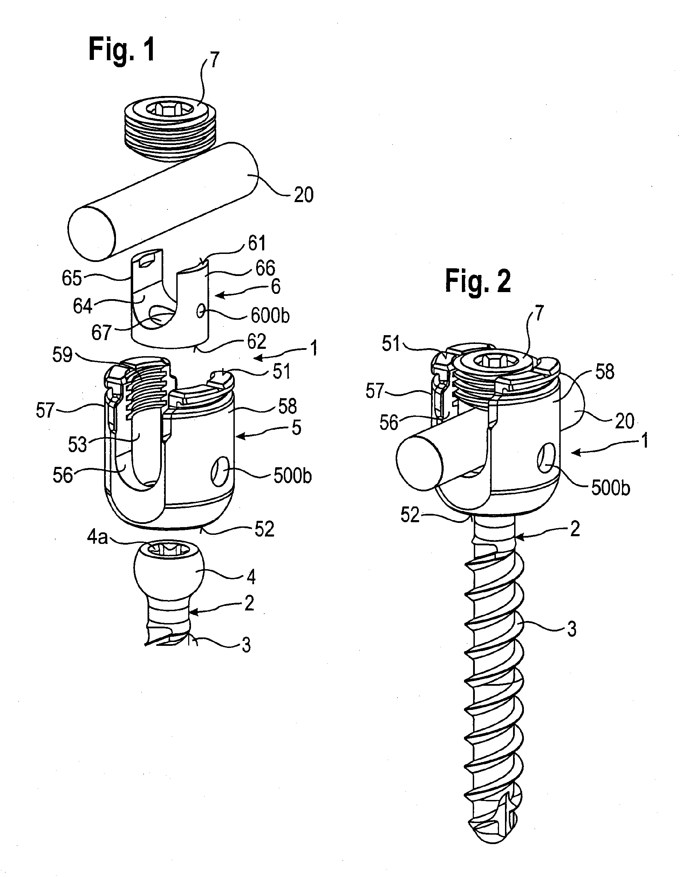

The polyaxial bone anchoring device 1 according to a first embodiment as shown in Figs. 1 to 3 includes a bone anchoring element in the form of a screw member 2 having a threaded shaft 3 and a head 4. The head 4 is generally spherical and includes a recess 4a at its free end for engagement with a tool to insert the threaded shaft 3 into bone. The bone anchoring device further includes a receiving part 5 for connecting the screw member 2 to a rod 20. A pressure element 6 is arranged in the receiving part on top of the head 4. For securing the rod 20 in the receiving part and for exerting pressure onto the head, a locking device, for example an inner screw 7, which cooperates with the receiving part 5, is provided.

The receiving part is a substantially cylindrical one piece part and has a top end 51 and a bottom end 52. A passageway extending from the top end to the bottom end is formed by a coaxial bore 53 followed by a seat portion 54 for receiving the head 4 of the screw member 2. The seat portion 54 has an opening 55 at the bottom end 52 through which the shaft 3 of the screw member extends. The seat portion 54 is shown to be spherically-shaped, but it can be tapered or it can have any other shape that allows to receive the head 4 so that it can pivot with respect to the receiving part 5. At the top end 51 a substantially U-shaped recess 56 is provided by means of which two free legs 57, 58 are formed that are the sidewalls of a channel for receiving the rod 20. An internal thread 59 is provided at the legs for cooperating with the inner screw 7.

The pressure element 6 is formed in one piece. It is of substantially cylindrical construction and has an outer diameter, which allows it to move in the axial direction within the bore 53 of the receiving part 5. The pressure element 6 has a top end 61 and a bottom end 62. When the pressure element is inserted into the receiving part, the bottom end 62 faces the head 4 of the screw element 2. At the bottom end 62 a spherical recess 63 is provided, which is adapted to the size and shape of the head 4. The spherical recess is configured to come into frictional engagement with the spherical surface of the head. At the top end 61, a U-shaped recess 64 is provided by means of which two free legs 65, 66 are formed that form a channel to receive the rod 20 therein. Furthermore, the pressure element 6 includes a coaxial bore 67 for accessing the screw head 4 with a tool (not shown). As shown in the Figs., the pressure element 6 is a solid member without any spring portions, which could render it flexible. It is arranged in the receiving part such that the U-shaped recess 56 of the receiving part 5 and the U-shaped recess 64 of the pressure element are aligned.

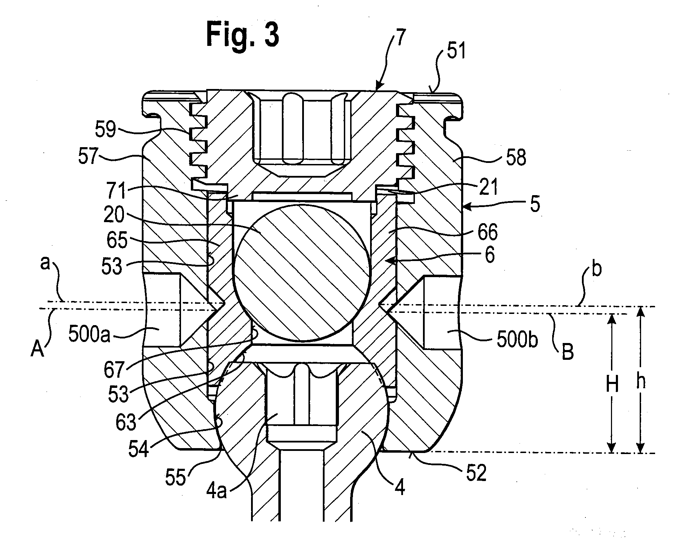

In the assembled state as shown in Fig. 3, the screw head 4 is located in the seat 54 and the pressure element 6 is arranged on top of the screw head 4. The height of the free legs 65, 66 of the pressure element is configured such that the free legs 65, 66 extend above the rod 20 when the rod is inserted and rests on the bottom of the channel.

The locking device in the form of the inner screw 7 has a projection 71 extending into the channel formed by the free legs 65, 66 of the pressure element 6. The size of the projection 71 in an axial direction is such that when the inner screw 7 is tightened, the projection 71 presses onto the rod while there is still a gap 21 between the top end 61 of the pressure element and the lower side of the inner screw 7. Therefore, with the single inner screw 7 pressure can be exerted onto the rod 20 only, which in turn can exert pressure onto the pressure element 6. It should be noted that instead of the single part locking device in form of the inner screw 7 a two-part locking device can be used (not shown). The two-part locking device includes a first part to be screwed in-between the legs 57, 58 of the receiving part. The first part acts onto the top end 61 of the pressure element 6. Further, a second part in form of an inner screw is provided in the first part, which presses onto the rod 20. By means of this, the head 4 and the rod 5 can be independently fixed.

The pressure element 6 is retained in the receiving part 5 as shown in Figs. 3 to 5. As shown in particular in 4a and 4b, the receiving part includes two blind holes 500a, 500b forming crimp bores that extend from the outer surface to a distance from the inner wall of the coaxial bore 53. The blind holes 500a, 500b are arranged at 180° offset from each other and at 90° with respect to the channel formed by the U-shaped recess 56. The blind holes 500a, 500b are aligned perpendicular with respect to the bore axis M of the coaxial bore 53. At their end they are tapered with an angle α preferably less than 45°, for example 22,5°, with respect to an axis parallel to the bore axis M. The bore axes A and B of the blind holes 500a, 500b are provided at a distance H from the second end 52 of the receiving part.

The portions of the receiving part that are between the closed ends of the blind holes 500a, 500b and the coaxial bore 53 of the receiving part are configured to be deformable portions 501a, 501b.

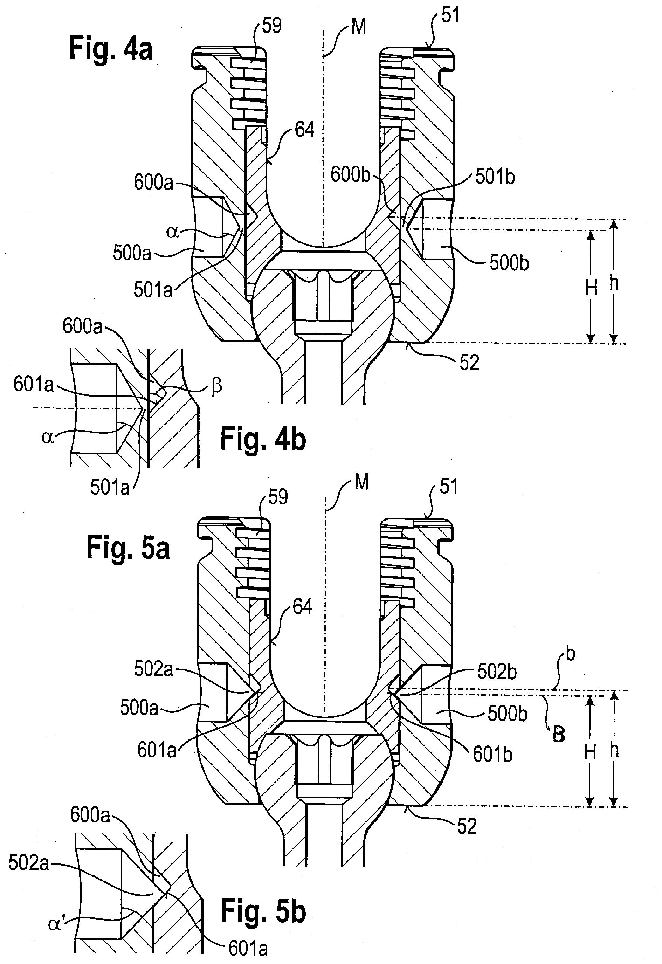

The pressure element 6 correspondingly includes two recesses 600a, 600b which are 180° offset from each other and 90° offset from the channel formed by the U-shaped recess 64. The recesses 600a, 600b have a center axis a, b, respectively, which is perpendicular to the bore axis M. In the embodiment shown, the recesses 600a, 600b have a conical shape. The downwardly extending flanks 601a, 601b of the recesses 600a, 600b each include an angle β of approximately 45° with the central bore axis M. As shown in Fig. 4a and 4b, when the pressure element 6 is inserted such that it rests on the head 4 of the screw element, the central axis a, b of the recesses 600a, 600b has a distance h from the second end 52 of the receiving part 5 that is greater than the distance H of the central axis A, B of the blind holes 500a, 500b. In other words, the recesses 600a, 600b are arranged above the blind holes 500a, 500b.

The distance between the recesses and the blind holes is such that when the deformable portions 501a, 501b are deformed by applying a force via for example, a crimping tool to the blind holes 500a, 500b, the deformed material protrudes from the inner wall of the receiving part and presses onto the lower flanks 601a, 601b of the recesses 600a, 600b, respectively, to exert a downward force onto the pressure element 6. As shown in Figs. 5a and 5b, deforming the deformable portions 5a, 5b results in deformed portions 502a, 502b which exert pressure on the lower flank 601a, 601b of the recesses 600a, 600b of the pressure element 6. For example, after deformation, the angle α is approximately 45°, which is approximately the same as the angle of the lower flank 601a, 601b. The blind holes 500a, 500b with their respective deformable portions 501a, 501b and the recesses 600a, 600b are constructed such that by deforming the deformable portions 501a, 501b into deformed portions 502a, 502b which engage the recesses 600a, 600b, the resulting force onto the pressure element 6 generates a preload onto the head 4, which clamps the head by means of friction. By selecting the sizes of blind holes and the recesses and their position, a desired friction force can be achieved. By this friction force the head can be maintained in a desired angular position and can be moved out of this position by applying a force greater than the friction force either to the screw element or to the receiving part. Simultaneously, the pressure element is secured against rotation and secured against escaping through the top end 51 of the receiving part. The recesses 600a, 600b provide space for accommodating a part of the deformed material. Also, the recesses 600a, 600b provide space for the protrusions 502a, 502b when the pressure element 6 moves downward to finally lock the head.

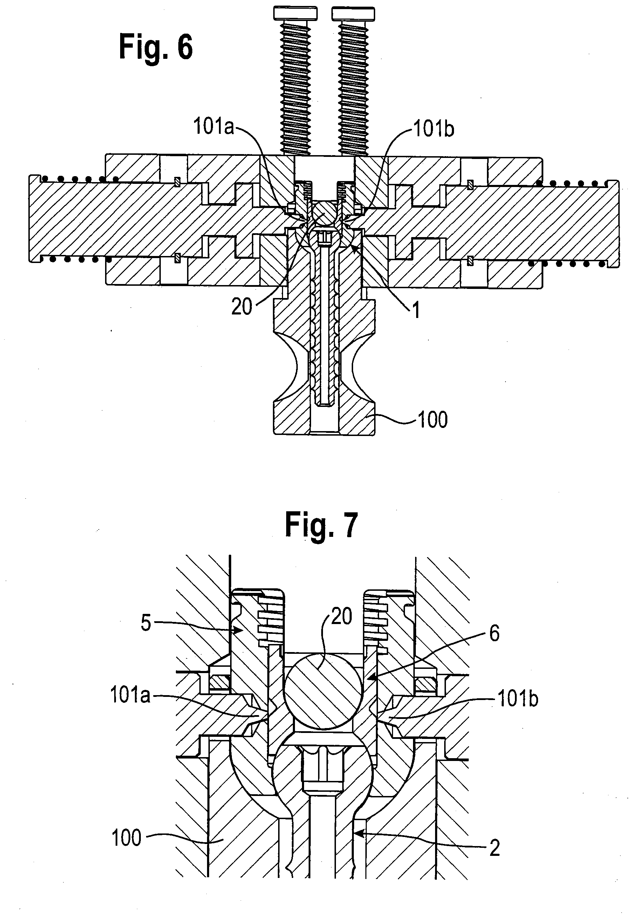

A method for manufacturing the polyaxial bone anchoring device is explained with reference to Figs. 6 and 7 and 4 to 5. A crimping tool shown in Figs. 6 and 7 generally comprises a holder 100 for the bone anchoring device, which serves for fixing the receiving part 5 with inserted screw element 2 and pressure element 6 as it is shown in Figs. 4a and 4b. The rod 20 may be inserted for providing a counter-force to avoid deformation of the free legs 65, 66 of the pressure element. The crimping tool further includes two crimping tips 101a, 101b, which are arranged 180° offset from each other and are dimensioned to be introduced into the blind holes 500a, 500b and to deform the deformable portions 501a, 501b, so that the displaced material, which forms the deformed portions 502a, 502b, engages the recesses 600a, 600b of the pressure element. As can be seen in particular in Fig. 7, the crimping tips 101a, 101b have an angle, which is more acute than that of the bottom of the blind hole 500a, 500b. The crimping tips 101a, 101b deform the deformable portion such that the deformed portion presses onto the lower flank 601a, 601b of the recess 600a, 600b, respectively. Thereafter, the crimping tips are retracted. The crimping process can be force-actuated and/or path-controlled.

After the crimping tips are retracted, the polyaxial anchoring device can be removed from the holder 100. The polyaxial bone anchoring device is then in a pre-assembled state with the screw element 2 being inserted and the pressure element being held in such a way that it exerts a slight preload onto the head which frictionally holds the head in an angular position.

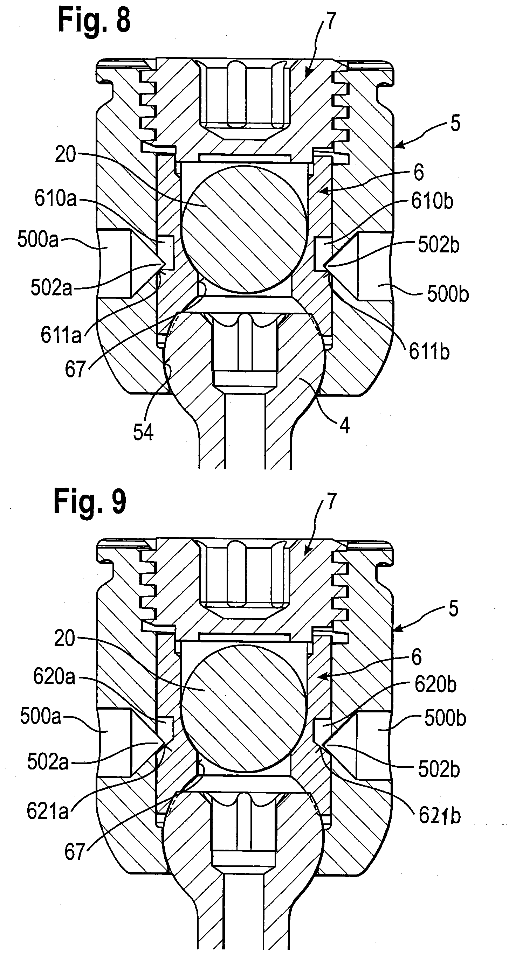

It shall be noted that the shape of the blind holes may vary. In particular, the angle of the conical bottom may vary or the bottom may have rounded or other shape. The recesses provided at the pressure element 6 may also have a different shape. As shown in Fig. 8, the recesses 610a, 610b can have, for example, a substantially rectangular cross-section. A lower side of the recess comprises an inclined edge 611a, 611b for engagement with the deformed portions 502a, 502b.

As shown in Fig. 9, the cross-section of the recesses 620a, 620b of the pressure element can be for example trapezoidal, with an inclined lower flank 621a, 621b for engagement with the deformed portions 502a, 502b.

All parts of the bone anchoring device are made of a body-compatible material, such as a body-compatible metal, for example, titanium, body-compatible metal alloys such as, for example, Nitinol or from a body-compatible plastic material, such as, for example, polyether ether ketone (PEEK) or combinations thereof.

Usually, several bone anchoring devices are necessary for stabilizing bone parts or vertebrae with the rod. In use, the bone anchoring devices are pre-assembled as shown in Fig. 5a, 5b. The screw members are screwed into the bone or a vertebra. Then, the receiving parts are pivoted by applying a force greater than the friction force until each receiving part has the correct orientation for the insertion of the rod. Due to the friction force, each receiving part is held in this angular position. Thereafter, the rod, which connects the bone anchoring devices, is inserted and the inner screw is tightened to move the pressure element downwards to lock the head in the seat so that the angular position of the screw member with respect to the receiving part is fixed. The rod is simultaneously fixed by the inner screw. Since the deformed portions 502a, 502b engage only the lower flank of the recesses provided at the pressure element, the recesses provide enough space for the deformed portions to allow a downward movement of the pressure element.

Further modifications of the previously described embodiment are conceivable. For example, only one deformed portion at the receiving part and one corresponding recess at the pressure element is sufficient. However, more than two deformed portions and corresponding recesses can also be provided.

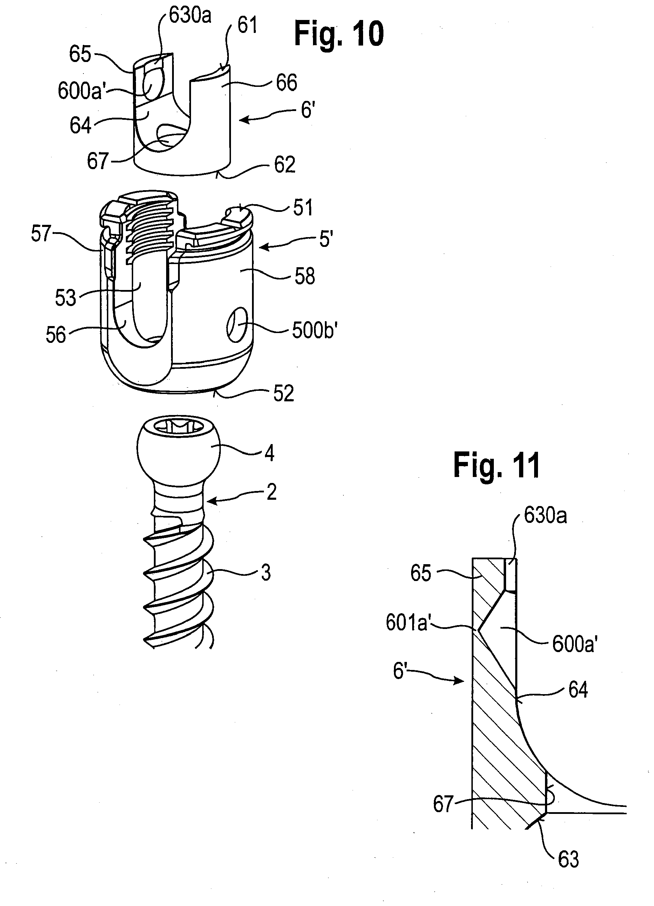

A second embodiment of the bone anchoring device is described with reference to Figs. 10 to 13. Parts or portions that are identical or similar to the previously decribed embodiment are designated with the same reference numerals and the decription thereof will not be repeated. The second embodiment differs from the first embodiment mainly in that the functions of the pressure element and the receiving part with respect to the provisional fixation with preload onto the head are reversed.

As can be seen in Fig. 9, the receiving part 5' has instead of the blind holes 500a, 500b two through holes 500a', 500b'. Although recesses at the inner wall of the receiving part instead of through holes would be sufficient, providing through holes is easier to manufacture and allows to upgrade existing receiving parts that have the blind holes of the first embodiment.

The pressure element 6' has two recesses 600a', 600b', arranged offset by 180°, that extend from the inner wall of the channel 64 into the legs 65, 66, respectively. The recesses can have a substantially triangular cross-section with a taper of approximately 22,5° similar to that of the blind holes 500a, 500b of the receiving part of the first embodiment. At the upper edge of the recesses a rectangular recess 630a, 630b is provided, respectively, the depth of which is smaller than that of the recesses 600a', 600b'. The recesses 630a, 630b are optional and may facilitate the insertion of a crimping tool.

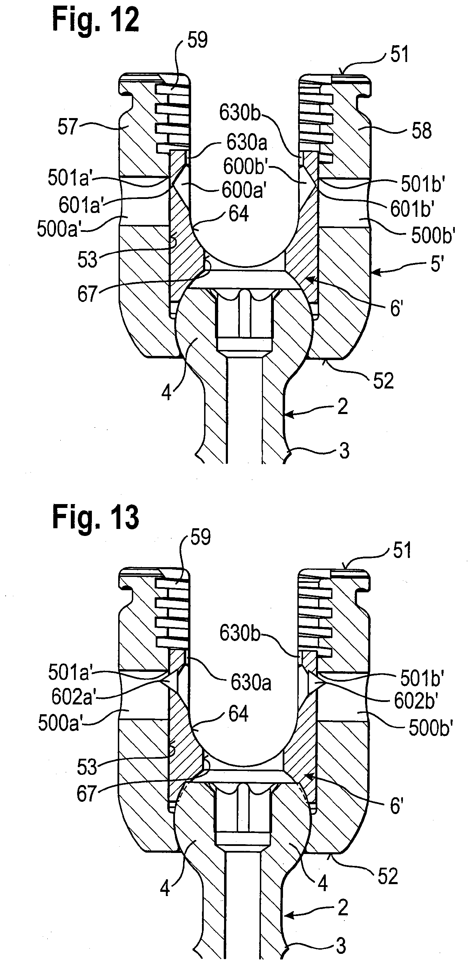

Between the outer surface of the pressure element 6' and the bottom of the recesses 600a', 600b', deformable portions 601a', 601b' are formed that can be deformed into deformed portions 602a', 602b' as shown in Fig. 13. In the pre-assembled and non-deformed state, as shown in Fig. 12, the pressure element 6' is situated in the receiving part 5' in such a position that it rests on the head 3 and the deformable portions are slightly below the upper wall portion of the through holes 500a', 500b'. Then, crimping tips (not shown) are introduced into the recesses 600a', 600b' and the deformable portions 601a', 601b' are deformed towards the outside. the deformed portions 602a', 602b' abut against the upper wall portion (501a', 501b') of the through holes 500a', 500b' at the inner side of the receiving part 5' as shown in Fig. 13. The deformed portions 602a', 602b' have then a taper of around 45°. When the deformed portions abut against the upper wall portion (501a', 501b') of the through holes, a downward force is exerted onto the head which clamps the head by friction.

The shape of the recesses and blind holes of the embodiments described is not limited to the tapered form. Also the angles of the taper are not limited to the values described. Other shapes are possible that achieve also a downwardly directed force when the deformable portions are deformed.

For the anchoring element, all kinds of anchoring elements can be used and combined with a receiving part. These anchoring elements are e.g. screws of different lengths, with different diameters, cannulated screws, screws with different thread forms, nails, hooks, etc. The head and the shaft can be separate parts which are connectable to each other.

The shape of the receiving part is not limited to the embodiment shown. For example, the receiving part can have an asymmetric end portion for allowing a greater pivot angle of the screw member to one side. Also, it is possible to have a recess allowing the rod to be introduced from the side instead of being introduced from the top or a closed recess through which the rod has to be guided. Various kinds of locking devices, including two- or more-part locking devices, outer nuts, outer caps, bayonet locking devices or others are possible.

In a further modification, the receiving part is configured to allow the introduction of the screw element from the bottom end.

## Claims

1. A polyaxial bone anchoring device including
an anchoring element (2) having a shaft (3) for anchoring in a bone and a head (4);
a receiving part (5) having a top end (51) and a bottom end (52), a channel (56) for receiving a rod therein and a passage (53, 54, 55) extending from the top end (51) to the bottom end (52) and including a seat (54) for receiving the head (4);
a pressure element (6) which is arranged in the passage (53) and configured to exert pressure onto the head (4), wherein the head is pivotable with respect to the receiving part (5) and can be locked at an angle by means of the pressure element (6);
the pressure element (6) comprising at least one recess (600a, 600b; 610a, 610b; 620a, 620b) facing the inner wall of the receiving part;
the receiving part (5) including at least one deformable portion (501a, 501b) provided at its inner wall, which upon deformation engages the recess (600a, 600b; 610a, 610b; 620a, 620b) at the pressure element;
wherein the deformable portion (502a, 502b) and the recess (600a, 600b; 610a, 610b; 620a, 620b) are arranged such that by engagement of the deformable portion with the recess a force is exerted by the pressure element (6) onto the head that maintains the head at an angular position by friction before locking the head,
**characterized in that** when the pressure element (6) is inserted into the receiving part (5) and rests upon the head (4), the recess (600a, 600b; 610a, 610b; 620a, 620b) has a lower edge (601a, 601b, 611a, 611b; 621a, 621b) that is located above the deformable portion (501a, 501b) in an axial direction (M) towards the top end (51), wherein the distance in the axial direction (M) between the lower edge of the recess and the deformable portion (501a, 501b) is such that the deformed portion exerts a downward pressure onto the pressure element, when engaging the lower edge (601a, 601b; 611a, 611b; 621a, 621b) of the recess to generate a defined friction force between the head and the pressure element.

2. A polyaxial bone anchoring device including
an anchoring element (2) having a shaft (3) for anchoring in a bone and a head (4);
a receiving part (5') having a top end (51) and a bottom end (52), a channel (56) for receiving a rod therein and a passage (53, 54, 55) extending from the top end (51) to the bottom end (52) and including a seat (54) for receiving the head (4);
a pressure element (6') which is arranged in the passage (53) and configured to exert pressure onto the head (4), wherein the head is pivotable with respect to the receiving part (5') and can be locked at an angle by means of the pressure element (6');
the pressure element (6') comprising a deformable portion (601a', 601b') at its outer wall;
the receiving part (5') including a recess (500a', 500b') open at least to its inner wall; wherein the deformable portion (601a', 601b') and the recess (500a', 500b') are arranged such that by engagement of the deformable portion with the recess a force is exerted by the pressure element (6') onto the head that maintains the head at an angular position by friction before locking the head,
**characterized in that** when the pressure element (6') is inserted into the receiving part (5') and rests upon the head (4), the recess (500a', 500b') has an upper edge (501a', 501b') that is located above the deformable portion (601a', 601b') in an axial direction (M) towards the top end (51), wherein the distance in the axial direction (M) between the upper edge of the recess and the deformable portion (601a', 601b') is such that the deformed portion exerts a downward pressure onto the pressure element, when engaging the upper edge (501a', 501b') of the recess to generate a defined friction force between the head and the pressure element.

3. The polyaxial bone anchoring device of claim 1 or 2, wherein the deformable portion (501a, 501b, 601a', 601b') is configured to plastically deform into a deformed portion (502a, 502b, 602a', 602b') extending into the passage (53).

4. The polyaxial bone anchoring device according to claim 1 or claim 3, wherein the receiving part includes a blind hole (500a, 500b) extending from the outer wall of the receiving part towards the inner wall and wherein the deformable portion (501a, 501b) is arranged between the inner wall and the blind hole.

5. The polyaxial bone anchoring device according to claim 1 or one of claims 3 to 4, wherein the deformable portion (501a, 501b) is deformable into a tapered protrusion extending from the inner wall of the receiving part into the passage.

6. The polyaxial bone anchoring device of claim 4 or 5, wherein a closed end of the blind hole is tapered and includes an angle (β) with the central axis (M) of the passage of less than 45°, preferably of around 22,5°.

7. The polyaxial bone anchoring device according to claim 1 or one of claims 3 to 6, wherein the recess (600a, 600b; 610a, 610b; 620a, 620b) has an inclined lower edge (601a, 611a, 621a), which includes an angle with the central axis (M) of the passage of preferably around 45°.

8. The polyaxial bone anchoring device according to claim 1 or one of claims 3 to 7, wherein the recesses (600a, 600b) provide space for the deformed portion (502a, 502b) in an axial direction towards the first end (51).

9. The polyaxial bone anchoring device according to claim 1 or one of claims 3 to 8, wherein at least two deformable portions (501a, 501b) and corresponding recesses (600a, 600b; 610a, 610b; 620a, 620b) are provided, which are offset by 180° in a circumferential direction.

10. The polyaxial bone anchoring device of claim 2 or 3, wherein the deformable portion (601a', 601b') is provided at the pressure element (6') which upon deformation engages an upper edge (501a', 501b') of the recess (500a', 500b') provided at the receiving part (5').

11. The polyaxial bone anchoring device of one of claims 1 to 10, wherein the pressure element (6, 6')) includes a top end (61) and a bottom end (62), a coaxial bore (67), a spherical recess (63) at the bottom end and a cylindrical or U-shaped recess (64) at the top end and wherein the cylindrical or U-shaped recess (64) is aligned with the recess (56) of the receiving part.

## Patentansprüche

1. Eine polyaxiale Knochenverankerungsvorrichtung, umfassend
ein Verankerungselement (2) mit einem Schaft (3) zum Verankern in einem Knochen sowie mit einem Kopf (4);
ein Aufnahmeteil (5) mit einem oberen Ende (51) und einem unteren Ende (52), einem Kanal (56) zum Aufnehmen eines Stabs darin und einem Durchgang (53, 54, 55), der sich von dem oberen Ende (51) zu dem unteren Ende (52) erstreckt und einen Sitz (54) zum Aufnehmen des Kopfes (4) aufweist;
ein Druckelement (6), welches in dem Durchgang (53) angeordnet und eingerichtet ist, Druck auf den Kopf (4) auszuüben, wobei der Kopf in Bezug auf das Aufnahmeteil (5) schwenkbar ist und bei einem Winkel mithilfe des Druckelements (6) gesperrt werden kann;
das Druckelement (6) umfasst wenigstens eine Ausnehmung (600a, 600b; 610a, 610b; 620a, 620b), die der Innenwand des Aufnahmeteils zugewandt ist;
das Aufnahmeteil (5) weist wenigstens einen verformbaren Abschnitt (501a, 501b) auf, der an seiner Innenwand vorgesehen ist, und welcher bei einer Verformung in die Ausnehmung (600a, 600b; 610a, 610b; 620a, 620b) an dem Druckelement eingreift;
wobei der verformbare Abschnitt (502a, 502b) und die Ausnehmung (600a, 600b; 610a, 610b; 620a, 620b) derart angeordnet sind, dass bei einem Eingriff des verformbaren Abschnitts in die Ausnehmung eine Kraft von dem Druckelement (6) auf den Kopf ausgeübt wird, die den Kopf bei einer Winkelposition durch Reibung vor dem Sperren des Kopfes festhält,
**dadurch gekennzeichnet, dass**, wenn das Druckelement (6) in das Aufnahmeteil (5) eingesetzt ist und auf dem Kopf (4) ruht, die Ausnehmung (600a, 600b; 610a, 610b; 620a, 620b) eine untere Kante (601a, 601b, 611a, 611b; 621a, 621b) besitzt, die oberhalb des verformbaren Abschnitts (501a, 501b) in einer axialen Richtung (M) zu dem oberen Ende (51) hin positioniert ist, wobei der Abstand in der axialen Richtung (M) zwischen der unteren Kante der Ausnehmung und dem verformbaren Abschnitt (501a, 501b) dermaßen ist, dass der verformbare Abschnitt auf das Druckelement einen nach unten gerichteten Druck ausübt, wenn er die untere Kante (601a, 601b; 611a, 611b; 621a, 621b) der Ausnehmung angreift, um eine bestimmte Reibungskraft zwischen dem Kopf und dem Druckelement zu erzeugen.

2. Eine polyaxiale Knochenverankerungsvorrichtung, umfassend
ein Verankerungselement (2) mit einem Schaft (3) zum Verankern in einem Knochen sowie mit einem Kopf (4);
ein Aufnahmeteil (5') mit einem oberen Ende (51) und einem unteren Ende (52), einem Kanal (56) zum Aufnehmen eines Stabs darin und einem Durchgang (53, 54, 55), der sich von dem oberen Ende (51) zu dem unteren Ende (52) erstreckt und einen Sitz (54) zum Aufnehmen des Kopfes (4) aufweist;
ein Druckelement (6'), welches in dem Durchgang (53) angeordnet und eingerichtet ist, Druck auf den Kopf (4) auszuüben, wobei der Kopf in Bezug auf das Aufnahmeteil (5') schwenkbar ist und bei einem Winkel mithilfe des Druckelements (6') gesperrt werden kann;
das Druckelement (6') umfasst einen verformbaren Abschnitt (601a', 601b') an seiner Außenwand;
das Aufnahmeteil (5') weist eine Ausnehmung (500a', 500b') auf, die zumindest zu seiner Innenwand hin offen ist;
wobei der verformbare Abschnitt (601a', 601b') und die Ausnehmung (500a', 500b') derart angeordnet sind, dass bei einem Eingriff des verformbaren Abschnitts in die Ausnehmung eine Kraft von dem Druckelement (6') auf den Kopf ausgeübt wird, die den Kopf bei einer Winkelposition durch Reibung vor dem Sperren des Kopfes festhält,
**dadurch gekennzeichnet, dass**, wenn das Druckelement (6') in das Aufnahmeteil (5') eingesetzt ist und auf dem Kopf (4) ruht, die Ausnehmung (500a', 500b') eine obere Kante (501a', 501b') besitzt, die oberhalb des verformbaren Abschnitts (601a', 601b') in einer axialen Richtung (M) zu dem oberen Ende (51) hin positioniert ist, wobei der Abstand in der axialen Richtung (M) zwischen der oberen Kante der Ausnehmung und dem verformbaren Abschnitt (601a', 601b') dermaßen ist, dass der verformbare Abschnitt auf das Druckelement einen nach unten gerichteten Druck ausübt, wenn er die obere Kante (501a', 501b') der Ausnehmung angreift, um eine bestimmte Reibungskraft zwischen dem Kopf und dem Druckelement zu erzeugen.

3. Die polyaxiale Knochenverankerungsvorrichtung gemäß Anspruch 1 oder 2, wobei der verformbare Abschnitt (501a, 501b, 601a', 601b') eingerichtet ist, sich in einen verformten Abschnitt (502a, 502b, 602a', 602b') plastisch zu verformen, der sich in den Durchgang (53) hinein erstreckt.

4. Die polyaxiale Knochenverankerungsvorrichtung gemäß Anspruch 1 oder Anspruch 3, wobei das Aufnahmeteil ein Blindloch (500 a, 500 b) aufweist, das sich von der Außenwand des Aufnahmeteils zu der Innenwand hin erstreckt, und wobei der verformbare Abschnitt (501a, 501b) zwischen der Innenwand und dem Blindloch angeordnet ist.

5. Die polyaxiale Knochenverankerungsvorrichtung gemäß Anspruch 1 oder einem der Ansprüche 3 bis 4, wobei der verformbare Abschnitt (501a, 501b) in einen sich verjüngenden Vorsprung verformbar ist, der sich von der Innenwand des Aufnahmeteils in den Durchgang hinein erstreckt.

6. Die polyaxiale Knochenverankerungsvorrichtung gemäß Anspruch 4 oder 5, wobei ein geschlossenes Ende des Blindlochs sich verjüngt und einen Winkel (β) mit der Mittenachse (M) des Durchgangs einschließt, der weniger beträgt als 45°, vorzugsweise ungefähr 22,5°.

7. Die polyaxiale Knochenverankerungsvorrichtung gemäß Anspruch 1 oder einem der Ansprüche 3 bis 6, wobei die Ausnehmung (600a, 600b; 610a, 610b; 620a, 620b) eine geneigte untere Kante (601a, 611a, 621a) besitzt, die einen Winkel mit der Mittenachse (M) des Durchgangs von ungefähr 45° einschließt.

8. Die polyaxiale Knochenverankerungsvorrichtung gemäß Anspruch 1 oder einem der Ansprüche 3 bis 7, wobei die Ausnehmungen (600a, 600b) einen Raum für den verformten Abschnitt (502a, 502b) in einer axialen Richtung hin zu dem ersten Ende (51) bereitstellen.

9. Die polyaxiale Knochenverankerungsvorrichtung gemäß Anspruch 1 oder einem der Ansprüche 3 bis 8, wobei zumindest zwei verformbare Abschnitte (501a, 501b) und entsprechende Ausnehmungen (600a, 600b; 610a, 610b; 620a, 620b) vorgesehen sind, die in einer Umfangsrichtung gegeneinander um 180° versetzt sind.

10. Die polyaxiale Knochenverankerungsvorrichtung gemäß Anspruch 2 oder 3, wobei der verformbare Abschnitt (601a', 601b') an dem Druckstück (6') vorgesehen ist, welcher bei der Verformung eine obere Kante (501a', 501b') der an dem Aufnahmeteil (5') vorgesehenen Ausnehmung (500a', 500b') angreift.

11. Die polyaxiale Knochenverankerungsvorrichtung gemäß einem der Ansprüche bis 10, wobei das Druckelement (6, 6') ein oberes Ende (61) und ein unteres Ende (62), eine koaxiale Bohrung (67), eine sphärisch Ausnehmung (63) an dem unteren Ende und eine zylindrische oder U-förmige Ausnehmung (64) an dem oberen Ende aufweist, und wobei die zylindrische oder U-förmige Ausnehmung (64) auf die Ausnehmung (56) des Aufnahmeteils ausgerichtet ist.

## Revendications

1. Dispositif d'ancrage d'os polyaxial comprenant :
un élément d'ancrage (2) ayant un arbre (3) pour s'ancrer dans un os et une tête (4) ;
une partie de réception (5) ayant une extrémité supérieure (51) et une extrémité inférieure (52), un canal (56) pour y recevoir une tige et un passage (53, 54, 55) s'étendant à partir de l'extrémité supérieure (51) jusqu'à l'extrémité inférieure (52) et comprenant un siège (54) pour recevoir la tête (4) ;
un élément de pression (6) qui est agencé dans le passage (53) et configuré pour exercer la pression sur la tête (4), dans lequel la tête peut pivoter par rapport à la partie de réception (5) et peut être bloquée à un arbre au moyen de l'élément de pression (6) ;
l'élément de pression (6) comprenant au moins un évidement (600a, 600b ; 610a, 610b ; 620a, 620b) faisant face à la paroi interne de la partie de réception ;
la partie de réception (5) comprenant au moins une partie déformable (501a, 501b) prévue au niveau de sa paroi interne qui, suite à la déformation, met en prise l'évidement (600a, 600b ; 610a, 610b ; 620a, 620b) au niveau de l'élément de pression ;
dans lequel la partie déformable (502a, 502b) et l'évidement (600a, 600b ; 610a, 610b ; 620a, 620b) sont agencés de sorte que, par la mise en prise de la partie déformable avec l'évidement, une force est exercée par l'élément de pression (6) sur la tête qui maintient la tête dans une position angulaire par friction avant le blocage de la tête,
**caractérisé en ce que** lorsque l'élément de pression (6) est inséré dans la partie de réception (5) et s'appuie sur la tête (4), l'évidement (600a, 600b ; 610a, 610b ; 620a, 620b) a un bord inférieur (601a, 601b ; 611a, 611b ; 621a, 621b) qui est positionné au-dessus de la partie déformable (501a, 501b) dans une direction axiale (M) vers l'extrémité supérieure (51), dans lequel la distance dans la direction axiale (M) entre le bord inférieur de l'évidement et la partie déformable (501a, 501b) est telle que la partie déformée exerce une pression descendante sur l'élément de pression, lors de la mise en prise du bord inférieur (601a, 601b ; 611a, 611b ; 621a, 621b) de l'évidement afin de générer une force de friction définie entre la tête et l'élément de pression.

2. Dispositif d'ancrage d'os polyaxial comprenant :
un élément d'ancrage (2) ayant un arbre (3) pour s'ancrer dans un os et une tête (4) ;
une partie de réception (5') ayant une extrémité supérieure (51) et une extrémité inférieure (52), un canal (56) pour y recevoir une tige et un passage (53, 54, 55) s'étendant à partir de l'extrémité supérieure (51) jusqu'à l'extrémité inférieure (52) et comprenant un siège (54) pour recevoir la tête (4) ;
un élément de pression (6') qui est agencé dans le passage (53) et configuré pour exercer la pression sur la tête (4), dans lequel la tête peut pivoter par rapport à la partie de réception (5') et peut être bloquée à un angle au moyen de l'élément de pression (6') ;
l'élément de pression (6') comprenant une partie déformable (601a', 601b') au niveau de sa paroi externe ;
la partie de réception (5') comprenant un évidement (500a', 500b') ouvert au moins sur sa paroi interne ;
dans lequel la partie déformable (601a', 601b') et l'évidement (500a', 500b') sont agencés de sorte que, par la mise en prise de la partie déformable avec l'évidement, une force est exercée par l'élément de pression (6') sur la tête qui maintient la tête au niveau d'une position angulaire par friction avant le blocage de la tête ;
**caractérisé en ce que** lorsque l'élément de pression (6') est inséré dans la partie de réception (5') et s'appuie sur la tête (4), l'évidement (500a', 500b') a un bord supérieur (501a', 501b') qui est positionné au-dessus de la partie déformable (601a', 601b') dans une direction axiale (M) vers l'extrémité supérieure (51), dans lequel la distance dans la direction axiale (M) entre le bord supérieur de l'évidement et la partie déformable (601a', 601b') est telle que la partie déformée exerce une pression descendante sur l'élément de pression, lors de la mise en prise du bord supérieur (501a', 501b') de l'évidement afin de générer une force de friction définie entre la tête et l'élément de pression.

3. Dispositif d'ancrage d'os polyaxial selon la revendication 1 ou 2, dans lequel la partie déformable (501a, 501b, 601a', 601b') est configurée pour se déformer plastiquement en une partie déformée (502a, 502b ; 602a', 602b') s'étendant dans le passage (53).

4. Dispositif d'ancrage d'os polyaxial selon la revendication 1 ou la revendication 3, dans lequel la partie de réception comprend un trou aveugle (500a, 500b) s'étenant à partir de la paroi externe de la partie de réception vers la paroi interne et dans lequel la partie déformable (501a, 501b) est agencée entre la paroi interne et le trou aveugle.

5. Dispositif d'ancrage d'os polyaxial selon la revendication 1 ou l'une des revendications 3 à 4, dans lequel la partie déformable (501a, 501b) est déformable en une saillie progressivement rétrécie s'étendant à partir de la paroi interne de la partie de réception dans le passage.

6. Dispositif d'ancrage d'os polyaxial selon la revendication 4 ou 5, dans lequel une extrémité fermée du trou aveugle est progressivement rétrécie et comprend un angle (β) avec l'axe central (M) du passage inférieur à 45°, de préférence d'environ 22,5°.

7. Dispositif d'ancrage d'os polyaxial selon la revendication 1 ou l'une des revendications 3 à 6, dans lequel l'évidement (600a, 600b, 610a, 610b, 620a, 620b) a un bord inférieur incliné (601a, 611a, 621a) qui comprend un angle avec l'axe central (M) du passage de préférence d'environ 45°.

8. Dispositif d'ancrage d'os polyaxial selon la revendication 1 ou l'une des revendications 3 à 7, dans lequel les évidements (600a, 600b) fournissent l'espace pour la partie déformée (502a, 502b) dans une direction axiale vers la première extrémité (51).

9. Dispositif d'ancrage d'os polyaxial selon la revendication 1 ou l'une des revendications 3 à 8, dans lequel on prévoit au moins deux parties déformables (501a, 501b) et les évidements (600a, 600b ; 610a, 610b ; 620a, 620b) correspondants qui sont décalés de 180° dans une direction circonférentielle.

10. Dispositif d'ancrage d'os polyaxial selon la revendication 2 ou 3, dans lequel la partie déformable (601a', 601b') est prévue au niveau de l'élément de pression (6') qui, suite à la déformation, met en prise un bord supérieur (501a', 501b') de l'évidement (500a', 500b') prévu au niveau de la partie de réception (5').

11. Dispositif d'ancrage d'os polyaxial selon la revendication l'une des revendications 1 à 10, dans lequel l'élément de pression (6, 6') comprend une extrémité supérieure (61) et une extrémité inférieure (62), un alésage coaxial (67), un évidement sphérique (63) au niveau d'une extrémité inférieure et un évidement cylindrique ou en forme de U (64) au niveau de l'extrémité supérieure et dans lequel l'évidement cylindrique ou en forme de U (64) est aligné avec l'évidement (56) de la partie de réception.
